(19) Europäisches Patentamt
European Patent Office
Office européen des brevets

(11) Publication number: **0 051 467**
**B1**

(12) **EUROPEAN PATENT SPECIFICATION**

(45) Date of publication of patent specification: **13.08.86**

(51) Int. Cl.⁴: **C 07 D 303/16**, C 07 C 69/63, C 07 D 301/00, C 07 C 67/287, A 61 K 31/335, A 61 K 31/215

(21) Application number: **81305161.2**

(22) Date of filing: **30.10.81**

(54) **Hexitol derivatives, their preparation and pharmaceutical compositions containing them.**

(30) Priority: **04.11.80 HU 264980**

(43) Date of publication of application:
**12.05.82 Bulletin 82/19**

(45) Publication of the grant of the patent:
**13.08.86 Bulletin 86/33**

(84) Designated Contracting States:
**AT BE CH DE FR GB IT LI NL SE**

(56) References cited:
**GB-A-1 490 649**

(73) Proprietor: **CHINOIN Gyogyszer és Vegyészeti Termékek Gyára RT.**
**To utca 1-5**
**H-1045 Budapest IV (HU)**

(72) Inventor: **Elekes, Ilona**
**Csata u. 16/a**
**H-1135 Budapest (HU)**
Inventor: **Institoris, Laszlo**
**Eotvos u. 32**
**H-1067 Budapest (HU)**
Inventor: **Medzihradszky, Kalman**
**Holdvilag u. 6**
**H-1118 Budapest (HU)**
Inventor: **Otvos, Laszlo**
**Boroka u. 13**
**H-1025 Budapest (HU)**
Inventor: **Medzihradszky, Hedvig**
**Holdvilag u. 6**
**H-1118 Budapest (HU)**
Inventor: **Di Gleria, Katalin**
**Kapy u. 25**
**H-1025 Budapest (HU)**
Inventor: **Sugar, Janos**
**Matroz u. 6**
**H-1035 Budapest (HU)**

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(72) Inventor: **Somfai-Relle, Zsuzsanna**
**Oktober 6. u. 16**
**H-1051 Budapest (HU)**
Inventor: **Eckhardt, Sandor**
**Julia u. 1**
**H-1026 Budapest (HU)**
Inventor: **Hidy, Ivan**
**Kelemen Laszlo u. 9**
**H-1026 Budapest (HU)**
Inventor: **Kerpel-Fronius, Sandor**
**Szirtes ut 16,**
**H-1016 Budapest (HU)**

(74) Representative: **Hildyard, Edward Martin et al**
**Frank B. Dehn & Co. Imperial House 15-19**
**Kingsway**
**London WC2B 6UZ (GB)**

**Description**

The present invention relates to hexitol derivatives, their preparation and pharmaceutical compositions containing them. The invention especially relates to hexitol derivatives containing free carboxy groups. The compounds of the present invention possess interesting tumour-inhibiting activity.

Certain derivatives of 3,4-diacylated dianhydrohexitols which contain carboxylate groups have been described in British patent specification No. 1,490,649. These hexitols are, however, difficult to convert into the corresponding free carboxylic acid derivatives either by alkaline or enzymatic hydrolysis, since the ester bonds attached directly to the hexitol skeleton are also susceptible to reaction under these reaction conditions.

The present invention is based on the discovery that the hexitols of the present invention which contain free carboxy groups possess an improved therapeutic index over corresponding known hexitols which are either unsubstituted or have only hydrophobic substituents.

Thus according to one feature of the present invention there is provided a hexitol of the general formula (I):

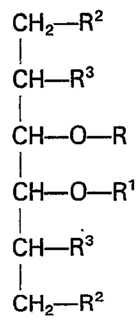

(I)

(wherein

the hexitol skeleton corresponds to dulcitol, mannitol or iditol;

$R^2$ represents a halogen atom,

$R^3$ represents a hydroxy group, or

$R^2$ and $R^3$ together form an oxygen atom;

R represents a free carboxy-containing alkylcarbonyl or alkenylcarbonyl group with 4 to 10 carbon atoms, a free carboxy-containing arylcarbonyl group with 8 to 12 carbon atoms or a free carboxy-containing aralkylcarbonyl or aralkenylcarbonyl group with 9 to 11 carbon atoms; and

$R^1$ represents a hydrogen atom, a free carboxy-containing alkylcarbonyl or alkenylcarbonyl group with 4 to 10 carbon atoms, a $C_{2-10}$ alkylcarbonyl or alkenylcarbonyl group, an alkoxycarbonyl-containing alkylcarbonyl or alkenylcarbonyl group with 4 to 10 carbon atoms, a $C_{8-10}$ aralkylcarbonyl or aralkenylcarbonyl group, a free carboxy-containing arylcarbonyl group with 8 to 12 carbon atoms or a free carboxy-containing aralkylcarbonyl or aralkenylcarbonyl group with 9 to 11 carbon atoms) or a salt thereof.

The carbon atom ranges given above and hereinafter include the C-atoms of any carboxy and alkoxycarbonyl substituents in the said groups. The term "free carboxy-containing" indicates that the groups in question contain one free carboxyl group.

The present invention thus relates to hexitols having the general formulae (Ia) and (Ib),

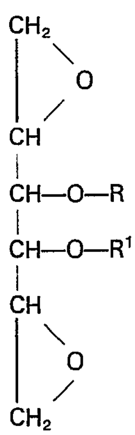

(Ia)

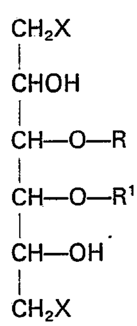

(Ib)

wherein

the hexitol skeleton is dulcitol, mannitol or iditol,

R and $R^1$ are as hereinbefore defined and

X represents halogen, preferably bromine.

The term "a free carboxy-containing $C_{4-10}$ alkylcarbonyl or alkenylcarbonyl group" refers to e.g. carboxybutyryl, carboxyvaleryl, carboxycapronyl, carboxycapryl, carboxypelargonyl, carboxyacrylyl, carboxycrotonyl and carboxymethacrylyl groups, of which the carboxypropionyl group is preferred.

The term "a $C_{2-10}$ alkylcarbonyl or alkenylcarbonyl group" refers to e.g. propionyl, butyryl, valeryl, capronyl, capryl, pelargonyl, caprinyl, acrylyl, crotonyl and methacrylyl groups. A preferred alkylcarbonyl group is acetyl.

Of the alkoxycarbonyl-containing alkylcarbonyl or alkenylcarbonyl groups with 4 to 10 carbon atoms, the following may for example be specifically mentioned: carbethoxypropionyl, carbopropoxypropionyl, carbobutoxypropionyl, carbopentoxypropionyl, carbohexyloxypropionyl, carbomethoxyacryl, carbomethoxycrotonyl and carbomethoxymethacryl groups. A preferred member of these groups is the carbomethoxypropionyl group.

The term "an aralkylcarbonyl or aralkenylcarbonyl group" refers for example to phenylacetyl, phenylbutyryl, phenylacryl, phenylcrotonyl and phenylpropionyl groups, of which phenylpropionyl is the most preferred.

Aryl groups include for example phenyl groups.

The salts of the compounds of formula (I) may for example be salts with alkali metals and alkaline earth metals, preferably lithium, potassium, sodium, calcium and magnesium salts. Salts of the compounds of formula (I) may also, for example, be formed with organic bases, preferably with amines, particularly with tris(hydroxymethylamino)-methane.

Compounds of the present invention thus include, for example, compounds of formula (I) in which R represents a free carboxy containing saturated or unsaturated alkylcarbonyl group with 4 to 10 carbon atoms; and

$R^1$ represents a hydrogen atom, a free carboxy-containing alkylcarbonyl or alkenylcarbonyl group with 4 to 10 carbon atoms, a $C_{2-10}$ alkylcarbonyl or alkenylcarbonyl group, an alkoxycarbonyl-containing alkylcarbonyl or alkenylcarbonyl group with 4 to 10 carbon atoms or a $C_{8-10}$ aralkylcarbonyl or aralkenylcarbonyl group.

Preferred compounds according to the present invention by virtue of their especially favourable properties include the following:—

1,2 - 5,6 - Dianhydro - 3 - (β - carboxypropionyl) - dulcitol or a salt thereof,

1,2 - 5,6 - Dianhydro - 3 - (β - carboxypropionyl) - 4 - acetyl - dulcitol or a salt thereof,

1,2 - 5,6 - Dianhydro - 3 - (β - carboxypropionyl) - 4 - (β - carbomethoxypropionyl) - dulcitol or a salt thereof,

1,2 - 5,6 - Dianhydro - 3 - (β - carboxypropionyl) - 4 - (β - phenylpropionyl) - dulcitol or a salt thereof,

1,6 - Dideoxy - 1,6 - dibromo - 3,4 - bis(β - carboxypropionyl) - dulcitol or a salt thereof,

1,2 - 5,6 - Dianhydro - 3 - (β - carboxypropionyl) - mannitol or a salt thereof, and

1,2 - 5,6 - Dianhydro - 3,4 - bis(β - carboxypropionyl) - mannitol or a salt thereof.

1,2 - 5,6 - Dianhydro - 3,4 - bis(β - carboxypropionyl) - dulcitol or a salt thereof is especially preferred.

It will be appreciated that the salts for pharmaceutical use are the physiologically compatible salts, but other salts may be useful in the preparation of the compounds of formula I and the physiologically compatible salts thereof.

According to a further feature of the present invention there are provided processes for the preparation of the compounds of the invention, which processes are selected from (a) to (c):—

a) for the preparation of a compound of formula (Ia) the hydrogenation of a compound of the general formula (IX),

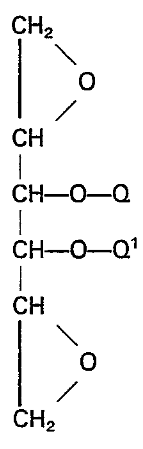

(IX)

(wherein the hexitol skeleton corresponds to dulcitol, mannitol or iditol, Q represents an aralkoxycarbonyl-containing $C_{3-9}$ alkylcarbonyl or alkenylcarbonyl group e.g. a benzyloxycarbonyl-containing alkylcarbonyl group with 11 to 17 carbon atoms and $Q^1$ represents a hydrogen atom, a $C_{2-10}$ alkylcarbonyl or alkenylcarbonyl group, an alkoxycarbonyl-containing alkylcarbonyl or alkenylcarbonyl group with 4 to 10 carbon atoms, an aralkoxycarbonyl-containing $C_{3-9}$ alkylcarbonyl or alkenylcarbonyl group e.g. a benzyloxycarbonyl-containing alkylcarbonyl group with 11 to 17 carbon atoms or a $C_{8-10}$ aralkylcarbonyl or aralkenylcarbonyl group) in the presence of a hydrogenation catalyst substantially ineffective to cause cleavage of the oxirane rings. The hydrogenation is preferably effected in the presence of a palladium/carbon catalyst, but if desired may be effected in the presence of any other weakly active catalyst i.e. a catalyst which is substantially ineffective to cause cleavage of the oxirane rings. The hydrogenation is advantageously effected in the presence of an anhydrous solvent.

The hydrogenation is preferably effected in the presence of about one or about two molar equivalents of hydrogen gas. The number of molar equivalents of hydrogen used will depend on whether the compound of formula (IX) contains an aralkoxycarbonyl e.g. a benzyloxycarbonyl substituent on the 3-moiety or on the 3- and 4-moiety.

b) for the preparation of a hexitol of formula (Ia), the reaction of a dulcitol, mannitol or iditol derivative of the general formula (X),

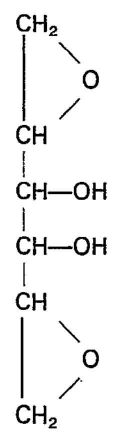

(X)

with a compound of the formula (XI),

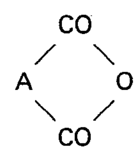

(XI)

(wherein A represents a $C_{2-10}$ alkyl, aryl or aralkyl group). The reaction is preferably effected in the presence of an anhydrous medium, conveniently in the presence of a base.

c) for the preparation of a compound of the formula (Ib), the reaction of a compound of formula (Ia) as hereinbefore defined with a halide preferably an alkali metal halide and/or a hydrogen halide.

If desired, a compound of general formula (I) may be converted into its salt.

The term "a benzyloxycarbonyl containing $C_{11-17}$ alkylcarbonyl or alkenylcarbonyl group refers to e.g. benzyloxycarbonyl-butyryl, benzyloxycarbonylvaleryl, benzyloxycarbonyl-caproyl, benzyloxycarbonyl-capryl, benzyloxycarbonyl-pelargonyl, benzyloxycarbonyl-acrylyl, benzyloxycarbonyl-crotonyl, benzyloxycarbonyl-methacrylyl and benzyloxycarbonyl-propionyl groups, of which benzyloxycarbonyl-propionyl is the most preferred.

Thus, for example, according to a preferred method of the invention 1,2 - 5,6 - dianhydro - 3,5 - bis(β - benzyloxycarbonyl - propionyl) - dulcitol of the formula (III),

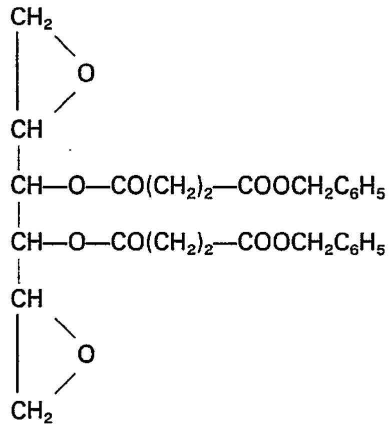

(III)

a compound prepared in known manner (see British patent specification No. 1,490,649), may be hydrogenated, for example in the presence of dry methanol and in the additional presence of a palladium-on-carbon catalyst conveniently until the uptake of the calculated amount (2 moles) of hydrogen. This generally requires about one or two hours. 1,2 - 5,6 - Dianhydro - 3,4 - bis - (β - carboxypropionyl) - dulcitol (which can also be termed 1,2 - 5,6 - dianhydro - 3,4 - disuccinyl - dulcitol) of formula (IV),

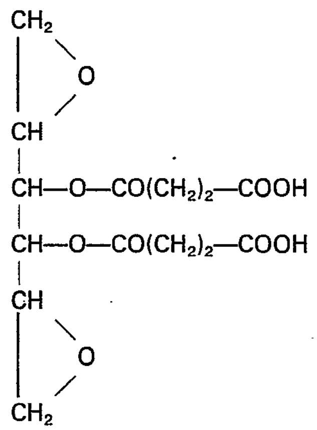

(IV)

may be crystallized out from a mixture of tetrahydrofuran and petroleum ether, and is obtained in excellent yield.

Hydrogenation may also be performed in the presence of other solvent media. When selecting the hydrogenation catalyst care should be taken regarding its activity; catalysts which are active enough to additionally cleave the oxirane rings should not be used in the process.

Similarly 1,2 - 5,6 - dianhydro - 3 - (β - carboxypropionyl) - dulcitol of the formula (V)

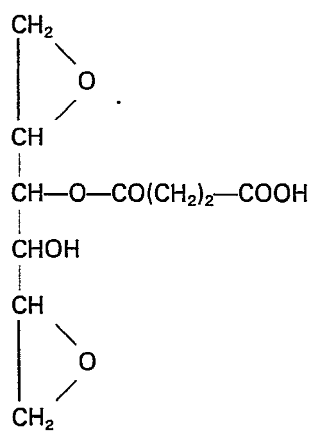

(V)

may, for example, be obtained from 1,2 - 5,6 - dianhydro - 3 - (β - benzyloxycarbonyl - propionyl) - dulcitol,

1,2 - 5,6 - dianhydro - 3 - (β - carboxypropionyl) - 4 - acetyl - dulcitol of the formula (VI)

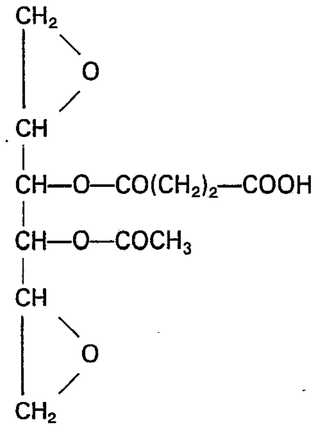

(VI)

may, for example, be obtained from 1,2 - 5,6 - dianhydro - 3 - (β - benzyloxycarbonyl - propionyl) - 4 - acetyl - dulcitol,

1,2 - 5,6 - dianhydro - 3 - (β - carboxypropionyl) - 4 - (β - carbomethoxypropionyl) - dulcitol of the formula (VII)

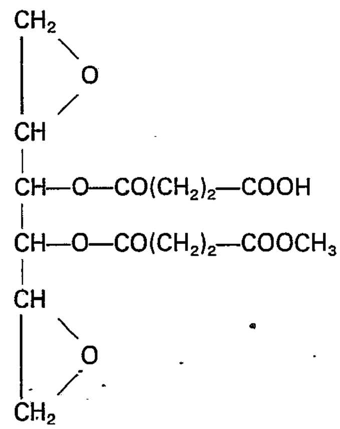

(VII)

may, for example, be obtained from 1,2 - 5,6 - dianhydro - 3 - (β - benzyloxycarbonyl - propionyl) - 4 - (β - carbomethoxy - propionyl) - dulcitol, and

1,2 - 5,6 - dianhydro - 3 - (β - carboxypropionyl) - 4 - (β - phenylpropionyl) - dulcitol of the formula (VIII)

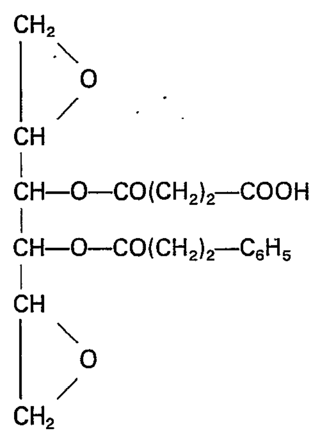

(VIII)

may, for example, be obtained from 1,2 - 5,6 - dianhydro - 3 - (β - benzyloxycarbonyl - propionyl) - 4 - (β - phenylpropionyl) - dulcitol.

The D-iditol and D-mannitol analogues of the D-dulcitol compounds having the formulae (IV) to (VIII) can be prepared similarly from the respective benzyl esters.

The β-carboxypropionyl derivatives of formula (Ia), for example may also be prepared according to the present invention by direct acylation of the corresponding dianhydro-hexitol with succinic anhydride. Thus, for example, a compound of formula (X), e.g. 1,2 - 5,6 - dianhydro - dulcitol, may be reacted

with succinic anhydride advantageously in the presence of an anhydrous solvent and preferably in the presence of a tertiary base. The product may be isolated from the reaction mixture in almost quantitative yield, and may then be subjected to column chromatography on silica gel to separate the monosuccinyl and disuccinyl compounds from each other. These compounds are identical to those obtained by the hydrogenolysis of the respective benzyl esters.

The dideoxy-dihalo derivatives of the above dianhydro compounds are prepared according to the invention by treating the dianhydro-hexitol compounds containing a free carboxy group with a halide, e.g. an alkali metal halide and/or a hydrogen halide, conveniently potassium bromide and hydrogen bromide. The dihalo compounds are crystalline substances easy to characterize and identify, and are generally formed in the reaction mixture in a yield of almost 100%.

The dianhydro-disuccinyl-hexitols prepared according to the invention are relatively poorly soluble in water, and this is so to an even greater extent for the dihalo-dideoxy-disuccinyl-hexitols. In order to increase their solubilities in water, these compounds may be converted into their salts, for example with alkali metals, alkaline earth metals and organic bases.

Compared to the known dianhydro-hexitols and dideoxy-dihalo-hexitols, which are either unsubstituted or have only hydrophobic substituents, the compounds of the present invention, which contain a free carboxy group, possess a more favourable therapeutic index. In order to illustrate this fact, the characteristic activity data of 1,2 - 5,6 - dianhydro - dulcitol (DAD) and 1,2 - 5,6 - dianhydro - 3,4 - diacetyl - dulcitol (diAcDAD), known from the literature, are compared with those of 1,2 - 5,6 - dianhydro - 3,4 - bis - (β - carboxypropionyl) - dulcitol (diSuDAD), a compound according to the invention. The data, observed on Walker i.m. carcinosarcoma, S-180 sarcoma and $P_{388}$ leukaemia, are listed in Table 1 below.

TABLE 1

| Compound | $LD_{50}$ mg/kg | $LD_{10}$ mg/kg | $LD_{10}$ μmol/kg | $ED_{90}$ μmol/kg | $LD_{10}$ / $ED_{90}$ |
|---|---|---|---|---|---|
| DAD | 15 | 10 | 68 | 16 | 4 |
| diAcDAD | 34 | 25.5 | 110 | 15 | 7 |
| diSuDAD | 630 | 190 | 550 | 14 | 40 |

The compounds of the present invention, especially the dianhydro-hexitols are of interest as tumour inhibitors and may be utilized in therapy in a number of ways.

According to a further feature of the present invention there are provided pharmaceutical compositions comprising as active ingredient at least one compound of formula I as hereinbefore defined or a physiologically compatible salt thereof in association with a pharmaceutical carrier or excipient.

The compounds of formula I and the physiologically compatible salts thereof may, for example, be converted into compositions for oral administration, e.g. tablets, either as such or in combination with carriers or excipients conventionally used in such compositions (e.g. starch, lactose, talc, etc.). Tablets for oral administration may contain e.g. 20 to 90% of the active ingredient.

The compounds may also for example be formulated into solutions or suspensions for injection purposes, e.g. utilizing water or a physiologically inert organic solvent such as various glycols, etc. The solutions, which may be administered e.g. as intravenous, intramuscular, intraperitoneal or intracavital injections, may contain e.g. 1 to 10% of active agent, whereas the suspensions, which may be administered e.g. as intramuscular, intraperitoneal or intracavital injections, may contain e.g. 1 to 70% of active agent.

The new compounds may also be employed in the form of compositions suitable for local application e.g. for the treatment of skin or operation sites, either alone or in combination with conventional antibacterial and/or wound-healing agents, such as sulfonamides, corticoids, vitamins, etc.

The pharmaceutical compositions according to the invention may contain a single active agent of the general formula (I) or a mixture of such active agents.

The invention is illustrated in the following non-limiting Examples, the Preparation merely illustrating the preparation of a palladium/charcoal catalyst which is used in certain of the Examples. All of the starting substances utilized in Examples 1 to 10 are known compounds.

Preparation

Preparation of a palladium/charcoal catalyst

a) 0.2 g of palladium chloride are dissolved in 2 ml of distilled 5.7 N hydrochloric acid whilst heating, the solution is then diluted, five-fold, with water and 0.9 g of activated charcoal (Merck, p.a.) added. The suspension is then boiled for some minutes. The suspension is cooled and then rendered alkaline (pH=9) with 20% aqueous sodium hydroxide solution whilst shaking, so that the solution above the solid becomes completely colourless.

b) 0.2 g of palladium chloride are dissolved in 2 ml of distilled 5.7 N hydrochloric acid whilst heating, the solution is diluted with 80 ml of distilled water, and 9.0 g of activated charcoal (Merck, p.a.) are added. The resulting suspension is rendered alkaline as described in (a) above.

The suspensions obtained according to (a) and (b) are mixed, blended thoroughly, the catalyst filtered off, washed chloride-free on the filter, and then washed with 200 ml of a 1% acetic acid solution. The wet catalyst is dried in a desiccator over concentrated sulfuric acid.

Example 1

Preparation of 1,2-5,6-dianhydro-3,4-bis(β-carboxypropionyl)-dulcitol

1.6 g (3 mmoles) of 1,2 - 5,6 - dianhydro - 3,4 - bis(β - benzyloxycarbonyl - propionyl) - dulcitol are dissolved in 150 ml of dry methanol, 0.3 g of a catalyst prepared according to Preparation 1 are added to the solution, and the resulting mixture is hydrogenated until completion of the uptake of the theoretical amount of hydrogen. This requires about 1.5 hours. The catalyst is filtered off, the solution is evaporated *in vacuo*, the resulting white, crystalline substance is suspended in ether, and the solids are filtered off. The substance is recrystallized from a mixture of ether and petroleum ether to obtain 1.0 g (96%) of 1,2 - 5,6 - dianhydro - 3,4 - bis(β - carboxypropionyl) - dulcitol; m.p.: 138—139°C. The product is uniform upon examination by layer chromatography ($R_f$=0.15; run with a 9:2 mixture of benzene and methanol and visualized with p-nitrobenzylpyridine).

Analysis:

| | | |
|---|---|---|
| calculated for $C_{14}H_{18}O_{10}$(346.29): | C: 48.78%, H: 5.73%, O: 45.49%; |
| found: | C: 48.35%, H: 5.25%, O: 46.60%. |

Equivalent weight: calculated: 173,
found: 180.

IR spectrum: 3350 $cm^{-1}$ (carboxy), 1235 $cm^{-1}$ (epoxy), benzyl and hydroxy bands do not appear.

Solubility: the compound is readily soluble in alcohol and tetrahydrofuran, but is poorly soluble in ether and water.

Example 2

Preparation of 1,2-5,6-dianhydro-3-(β-carboxypropionyl)-dulcitol

2.0 g (6 mmoles) of 1,2 - 5,6 - dianhydro - 3 - (β - benzyloxy - carbonyl - propionyl) - dulcitol are dissolved in 30 ml of dry methanol, 0.4 g of a palladium/charcoal catalyst (prepared as described in Preparation 1) are added, and the mixture is hydrogenated until completion of the uptake of the calculated amount (144 ml) of hydrogen. The catalyst is filtered off, the filtrate is evaporated *in vacuo*, and the solid residue recrystallized from a mixture of methanol and ether. 1.15 g (79%) of 1,2 - 5,6 - dianhydro - 3 - (β - carboxypropionyl) - dulcitol are obtained; m.p.: 77—78°C. The product is uniform as shown by examination by layer chromatography ($R_f$=0.1 or 0.5; run with a 6:4 mixture of ethyl acetate and cyclohexane or a 8:2 mixture of benzene and methanol and visualized with *p*-nitrobenzyl-pyridine).

Analysis:

| | |
|---|---|
| calculated for $C_{10}H_{14}O_7$ (246.22): | C: 48.78% H: 5.73% O: 45.49% |
| found: | C: 48.60% H: 5.33% O: 45.58%. |

Equivalent weight: calculated: 246,
found: 250.

IR spectrum: 3600—2950 $cm^{-1}$ (broad band referring to carboxy and hydroxy groups), 2940 $cm^{-1}$ (aliphatic CH), 1740 $cm^{-1}$ (ester), 1220 $cm^{-1}$ (epoxy).

Example 3

Preparation of 1,2-5,6-dianhydro-3-(β-carboxypropionyl)-4-acetyl-dulcitol

3.78 g (10 mmoles) of 1,2 - 5,6 - dianhydro - 3 - (β - benzyloxycarbonyl - propionyl) - 4 - acetyl - dulcitol are hydrogenated as described in Example 1 until completion of the uptake of hydrogen. The catalyst is filtered off, the filtrate is evaporated *in vacuo*, and the crystalline residue is recrystallized from a mixture of tetrahydrofuran and ethyl acetate. 2.5 g (88%) of 1,2 - 5,6 - dianhydro - 3 - (β - carboxypropionyl) - 4 - acetyl - dulcitol are obtained; m.p.: 134°C. The product is shown to be uniform on examination by layer chromatography ($R_f$=0.2 or 0.6; run with a 6:4 mixture of ethyl acetate and cyclohexane or a 8:2 mixture of benzene and methanol and visualized with *p* - nitrobenzylpyridine).

Analysis:

| | |
|---|---|
| calculated for $C_{12}H_{16}O_8$ (288.26): | C: 50.00%, H: 5.59%, O: 44.40%; |
| found: | C: 49.48%, H: 5.26%, O: 44.70%. |

Equivalent weight: calculated: 288,
found: 296.

Example 4

Preparation of 1,2-5,6-dianhydro-3-(β-carboxypropionyl)-4-(β-carbomethoxy-propionyl)-dulcitol

4.50 g (10 mmoles) of 1,2 - 5,6 - dianhydro - 3 - (β - benzyloxycarbonyl - propionyl) - 4 - (β - carbomethoxy - propionyl) - dulcitol are hydrogenated as described in Example 1 until completion of the uptake of the calculated amount of hydrogen. The catalyst is filtered off, and the solvent removed from the

filtrate. 3.1 g (86%) of crystalline 1,2 - 5,6 - dianhydro - 3 - (β - carboxypropionyl) - 4 - (β - carbomethoxy - propionyl) - dulcitol are obtained; m.p.: 87—89°C. The product is shown to be uniform upon examination by layer chromatography ($R_f$=0.25 or 0.4; run with a 6:4 mixture of ethyl acetate and cyclohexane or a 8:2 mixture of benzene and methanol).

Analysis:

| | | |
|---|---|---|
| calculated for $C_{15}H_{20}O_{10}$ (362.34): | C: 49.72%, H: 6.12%, O: 44.16%; |
| found: | C: 49.87%, H: 5.39%, O: 43.91%. |

Equivalent weight: calculated: 362,
found: 363.

Example 5

Preparation of 1,2-5,6-dianhydro-3-(β-carboxypropionyl)-4-(β-phenylpropionyl)-dulcitol

4.68 g (10 mmoles) of 1,2 - 5,6 - dianhydro - 3 - (β - benzyloxycarbonyl - propionyl) - 4 - (β - phenylpropionyl) - dulcitol are hydrogenated as described in Example 1. The catalyst is filtered off and the solvent removed from the filtrate to obtain 3.3 g (84%) of crystalline 1,2 - 5,6 - dianhydro - 3 - (β - carboxypropionyl) - 4 - (β - phenylpropionyl) - dulcitol, m.p.: 80°C. The product is shown to be uniform upon examination by layer chromatography ($R_f$=0.3 or 0.7; run with a 6:4 mixture of ethyl acetate and cyclohexane or a 8:2 mixture of benzene and methanol).

Analysis:

| | |
|---|---|
| calculated for $C_{19}H_{22}O_8$ (378.38): | C: 60.31%, H: 5.86%, O: 33.83%; |
| found: | C: 60.60%, H: 5.49%, O: 34.50%. |

Equivalent weight: calculated: 378;
found: 390.

Example 6

Preparation of 1,6-dideoxy-1,6-dibromo-3,4-bis(β-carboxypropionyl)-dulcitol

0.365 g (1 mmole) of 1,2 - 5,6 - dianhydro - 3,4 - bis(β - carboxypropionyl) - dulcitol are dissolved in 5 ml of methanol, and a solution of 1.2 g (10 mmoles) of potassium bromide in 5 ml of water is added. Thereafter a mixture of 1 ml of a 48% hydrobromic acid and 0.5 ml of water is added dropwise to the mixture over a period of 10 minutes with constant stirring. After stirring the mixture at room temperature for an additional half hour a clear solution is obtained, and after an additional 2 hours a white, crystalline substance starts to separate out. The separated substance is filtered off and washed with distilled water until neutral. 0.460 g (90%) of 1,6 - dideoxy - 1,6 - dibromo - 3,4 - bis(β - carboxypropionyl) - dulcitol are obtained; m.p.: 165°C. The product is shown to be uniform upon examination by layer chromatography ($R_f$=0.05; run with a 9:2 mixture of benzene and methanol and visualized with *p*-nitrobenzyl-pyridine).

Analysis:

| | |
|---|---|
| calculated for $C_{14}H_{20}O_{10}Br_2$ (508.13): | C: 33.09%, H: 3.97%, O: 31.49%, Br: 31.45%; |
| found: | C: 33.42%, H: 4.00%, O: 31.23%, Br: 31.50%. |

Equivalent weight: calculated: 254,
found: 256.

Example 7

Preparation of 1,2-5,6-dianhydro-3-(β-carboxypropionyl)-dulcitol and 1,2-5,6-dianhydro-3,4-bis(β-carboxypropionyl)-dulcitol by acylation of 1,2-5,6-dianhydro-dulcitol with succinic anhydride

1.46 g (10 mmoles) of 1,2 - 5,6 - dianhydro - dulcitol are dissolved in 20 ml of dry ethyl acetate, 5 ml of pyridine are added to the solution, and then 2.0 g (20 mmoles) of succinic anhydride are added in small portions to the mixture with constant stirring. The mixture is warmed to 45°C and stirred at this temperature for an additional 8 hours. The solvent is distilled off *in vacuo*, the residue is taken up in a mixture of ethyl acetate and water, and the resulting mixture is acidified cautiously, whilst cooling in ice, to give a pH of 4 to 5. The organic phase is separated off, washed with water, dried and evaporated. 2.5 g of a solid residue are obtained, which correspond to a quantitative yield when calculated as the monosuccinyl-dianhydro-dulcitol.

The resulting crude substance is subjected to chromatography on a silica gel column, applying a 1:1 v/v mixture of methanol and ethyl acetate as the eluting agent. The fractions which contain pure products are combined, the solution is evaporated *in vacuo*, and the resulting solid is recrystallized from a mixture of ether and petroleum ether and then from a mixture of methanol and ether. 1.8 g of 1,2 - 5,6 - dianhydro - 3 - (β - carboxypropionyl) - dulcitol and 0.4 g of 1,2 - 5,6 - dianhydro - 3,4 - bis(β - carboxypropionyl) - dulcitol are obtained. The physical constants of these compounds are identical with those of the products obtained according to Examples 1 and 2.

Example 8

Preparation of the TRIS-salt of 1,2-5,6-dianhydro-3,4-bis(β-carboxypropionyl)-dulcitol

3.46 g (10 mmoles) of 1,2 - 5,6 - dianhydro - 3,4 - bis(β - carboxypropionyl) - dulcitol are dissolved in 20 ml of 96% ethanol with gentle warming, the solution is cooled, and a solution of 1.2 g (10 mmoles) of tris(hydroxymethylamino)-methane in 10 ml of water is added. The mixture is allowed to stand in a

refrigerator, then the separated crystals are filtered off and dried in a desiccator. 3.7 g (80%) of the TRIS-salt are obtained.

Example 9

Preparation of 1,2-5,6-dianhydro-3,4-bis(β-carboxypropionyl)-mannitol

1.6 g (3 mmoles) of 1,2 - 5,6 - dianhydro - 3,4 - bis(β - benzyloxycarbonyl - propionyl) - mannitol are dissolved in 150 ml of tetrahydrofuran, 0.4 g of a catalyst (prepared as described in Preparation 1) are then added to the solution, and the mixture is hydrogenated until completion of the uptake of the calculated amount (288 ml) of hydrogen. The catalyst is filtered off, the solvent is distilled off *in vacuo*, and the solid residue is recrystallized from a mixture of ethyl acetate and petroleum ether. 0.85 g (82%) of 1,2 - 5,6 - dianhydro - 3,4 - bis(β - carboxypropionyl) - mannitol are obtained; m.p.: 125—130°C. The hygroscopic, crystalline substance is shown to be uniform when examined by layer chromatography ($R_f$=0.6; run in a 1:1 mixture of tetrahydrofuran and methanol).

Analysis:

| | | |
|---|---|---|
| calculated for $C_{14}H_{18}O_{10}$ (346.29): | C: 48.56%, H: 5.24%, O: 46.20%; |
| found: | C: 48.35%, H: 5.48%, O: 46.56%. |

Example 10

Preparation of 1,2-5,6-dianhydro-3-(β-carboxypropionyl)-mannitol

2 g (6 mmoles) of 1,2 - 5,6 - dianhydro - 3 - (β - benzyloxy - carbonyl - propionyl) - mannitol are dissolved in 30 ml of tetrahydrofuran, 0.4 g of a palladium/charcoal catalyst (prepared as described in Preparation 1) are added, and the mixture is hydrogenated until completion of the uptake of the calculated amount (144 ml) of hydrogen. The catalyst is filtered off and the solvent is evaporated *in vacuo* to obtain 1.13 g (78%) of 1,2 - 5,6 - dianhydro - 3 - (β - carboxypropionyl) - mannitol as a thick, viscous oil. The product is shown to be uniform when examined by layer chromatography ($R_f$=0.8; run in a 1:1 mixture of tetrahydrofuran and methanol.

Analysis:

| | |
|---|---|
| calculated for $C_{10}H_{14}O_7$ (246.22): | C: 48.78%, H: 5.73%; |
| found: | C: 48.26%, H: 5.96%. |

Equivalent weight: calculated: 246,
found: 238.

Example 11

Preparation of freeze-dried powder ampoules containing 10 mg of 1,2-5,6-dianhydro-3,4-bis(β-carboxypropionyl)-mannitol

One powder ampoule contains 10 mg of 1,2 - 5,6 - dianhydro - 3,4 - bis - (β - carboxypropionyl) - mannitol; one solvent ampoule contains 300 mg of sorbitol+distilled water for injection q.s. ad 2 ml.

The pharmaceutical compositions are prepared as follows:

50 g of pure (100%) 1,2 - 5,6 - dianhydro - 3,4 - bis(β - carboxypropionyl) - mannitol of pharmaceutical quality are filled into a calibrated vessel, 10 litres in volume. The active agent is dissolved in the vessel at 20°C in distilled water suitable for injection purposes, free of carbon dioxide, and the contents of the flask adjusted to the mark with distilled water of the same quality. Thereafter the solution is sterilized by filtration under aseptic conditions. 2 ml portions of the sterile solution are filled into ampoules and subjected to freeze-drying in a conventional industrial apparatus. The frozen substance is sublimated at a hypoeutectic temperature, and the final drying step is performed at 30°C. Thereafter the ampoules are stoppered with a rubber stopper, sealed with a metal band under sterile conditions, and then provided with an appropriate signature. The powder ampoules are packaged with solvent ampoules filled with 2 ml of a 15 w/v % aqueous sorbitol solution, prepared according to standard techniques.

Example 12

Preparation of capsules containing 100 mg of 1,2-5,6-dianhydro-3,4-bis(β-carboxypropionyl)-mannitol

One capsule contains 100 mg of 1,2 - 5,6 - dianhydro - 3,4 - bis(β - carboxypropionyl) - mannitol
5 mg of Carbowax 6000 and
5 mg of talc

Carbowax and talc are homogenized, the resulting fine, powdery homogenizate is mixed with the pure, crystalline active ingredient of pharmaceutical quality, and the resulting granules are filled into hard gelatine capsules. One capsule contains 110 mg of the granular mix.

Example 13

Preparation of intestinosolvent capsules

The capsules prepared according to Example 12 are provided with an intestinosolvent coating.

**Claims for the Contracting States: BE, CH, DE, FR, GB, IT, LI, NL, SE**

1. A hexitol of the general formula (I):

$CH_2$—$R^2$
|
CH—$R^3$
|
CH—O—R (I)
|
CH—O—$R^1$
|
CH—$R^3$
|
$CH_2$—$R^2$

wherein

the hexitol skeleton corresponds to dulcitol, mannitol or iditol;

$R^2$ represents a halogen atom,

$R^3$ represents a hydroxy group, or

$R^2$ and $R^3$ together form an oxygen atom;

R represents a free carboxy-containing alkylcarbonyl or alkenylcarbonyl group with 4 to 10 carbon atoms, a free carboxy-containing arylcarbonyl group with 8 to 12 carbon atoms or a free carboxy-containing aralkyl-carbonyl or aralkenylcarbonyl group with 9 to 11 carbon atoms; and

$R^1$ represents a hydrogen atom, a free carboxy-containing alkylcarbonyl or alkenylcarbonyl group with 4 to 10 carbon atoms, a $C_{2-10}$ alkylcarbonyl or alkenylcarbonyl group, an alkoxycarbonyl-containing alkylcarbonyl or alkenylcarbonyl group with 4 to 10 carbon atoms, a $C_{8-10}$ aralkylcarbonyl or aralkenyl-carbonyl group, a free carboxy-containing arylcarbonyl group with 8 to 12 carbon atoms or a free carboxy-containing aralkylcarbonyl or aralkenylcarbonyl group with 9 to 11 carbon atoms) or a salt thereof.

2. A hexitol as claimed in claim 1 wherein $R^2$ represents a bromine atom and $R^3$ represents a hydroxy group.

3. A hexitol as claimed in claim 1 wherein $R^2$ and $R^3$ together form an oxygen atom.

4. A hexitol as claimed in claim 1 which is 1,2 - 5,6 - dianhydro - 3 - (β - carboxypropionyl) - dulcitol or a salt thereof.

5. A hexitol as claimed in claim 1 which is 1,2 - 5,6 - dianhydro - 3 - (β - carboxypropionyl) - 4 - acetyl - dulcitol or a salt thereof.

6. A hexitol as claimed in claim 1 which is 1,2 - 5,6 - dianhydro - 3 - (β - carboxypropionyl) - 4 - (β - carbomethoxypropionyl) - dulcitol or a salt thereof.

7. A hexitol as claimed in claim 1 which is 1,2 - 5,6 - dianhydro - 3 - (β - carboxypropionyl) - 4 - (β - phenylpropionyl) - dulcitol or a salt thereof.

8. A hexitol as claimed in claim 1 which is 1,6 - dideoxy - 1,6 - dibromo - 3,4 - bis(β - carboxypropionyl) - dulcitol or a salt thereof.

9. A hexitol as claimed in claim 1 which is 1,2 - 5,6 - dianhydro - 3 - (β - carboxypropionyl) - mannitol or a salt thereof.

10. A hexitol as claimed in claim 1 which is 1,2 - 5,6 - dianhydro - 3,4 - bis(β - carboxypropionyl) - mannitol or a salt thereof.

11. A hexitol as claimed in claim 1 which is 1,2 - 5,6 - dianhydro - 3,4 - bis(β - carboxypropionyl) - dulcitol or a salt thereof.

12. A pharmaceutical composition comprising as active ingredient a hexitol of formula I as defined in any of claims 1 to 11 or a physiologically compatible salt thereof in association with a pharmaceutical carrier or excipient.

13. A pharmaceutical composition comprising as active ingredient 1,2 - 5,6 - dianhydro - 3,4 - bis(β - carboxy - propionyl) - dulcitol or a physiologically compatible salt thereof in association with a pharmaceutical carrier or excipient.

14. A process for the preparation of a hexitol as defined in claim 3, which comprises hydrogenating a compound of the formula (IX):

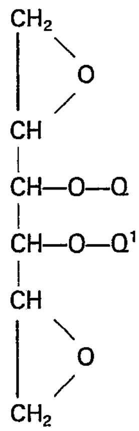

(IX)

(wherein the hexitol skeleton corresponds to dulcitol, mannitol or iditol, Q represents an aralkoxycarbonyl-containing $C_{3-9}$ alkylcarbonyl or alkenylcarbonyl group and $Q^1$ represents a hydrogen atom, a $C_{2-10}$ alkylcarbonyl or alkenylcarbonyl group, an alkoxycarbonyl-containing alkylcarbonyl or alkenylcarbonyl group with 4 to 10 carbon atoms, an aralkoxycarbonyl-containing $C_{3-9}$ alkylcarbonyl or alkenylcarbonyl group or a $C_{8-10}$ aralkylcarbonyl or aralkenylcarbonyl group) in the presence of a hydrogenation catalyst substantially ineffective to cause cleavage of the oxirane rings; or reacting a dulcitol, mannitol or iditol derivative of the formula (X):

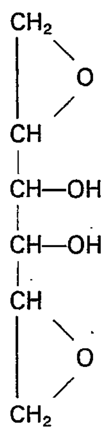

(X)

with a compound of the formula (XI):

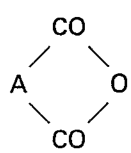

(XI)

wherein A represents a $C_{2-10}$ alkyl, aralkyl or aryl group.

15. A process as claimed in claim 14 wherein the hydrogenation catalyst comprises a palladium/carbon catalyst.

16. A process as claimed in claim 14 wherein said hydrogenating or reacting is effected in the presence of an anhydrous medium.

17. A process for the preparation of a hexitol as defined in claim 1 wherein $R^2$ represents a halogen atom and $R^3$ represents a hydroxy group, which comprises reacting a hexitol as defined in claim 3 with a halide.

18. A process as claimed in claim 17 wherein the halide comprises an alkali metal halide and/or a hydrogen halide.

**Claims for the Contracting State: AT**

1. A process for the preparation of a hexitol of the general formula (I):

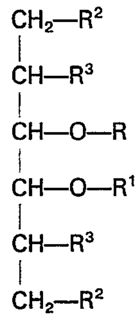

(I)

(wherein the hexitol skeleton corresponds to dulcitol, mannitol or iditol;

$R^2$ represents a halogen atom,

$R^3$ represents a hydroxy group, or

$R^2$ and $R^3$ together form an oxygen atom;

R represents a free carboxy-containing alkylcarbonyl or alkenylcarbonyl group with 4 to 10 carbon atoms, a free carboxy-containing arylcarbonyl group with 8 to 12 carbon atoms or a free carboxy-containing aralkylcarbonyl or aralkenylcarbonyl group with 9 to 11 carbon atoms; and

$R^1$ represents a hydrogen atom, a free carboxy-containing alkylcarbonyl or alkenylcarbonyl group with 4 to 10 carbon atoms, a $C_{2-10}$ alkylcarbonyl or alkenylcarbonyl group, an alkoxycarbonyl-containing alkylcarbonyl or alkenylcarbonyl group with 4 to 10 carbon atoms, a $C_{8-10}$ aralkylcarbonyl or aralkenylcarbonyl group, a free carboxy-containing arylcarbonyl group with 8 to 12 carbon atoms or a free carboxy-containing aralkylcarbonyl or aralkenylcarbonyl group with 9 to 11 carbon atoms) or a salt thereof; which comprises

(a) for the preparation of a hexitol wherein $R^2$ and $R^3$ together form an oxygen atom, hydrogenating a compound of the formula (IX):

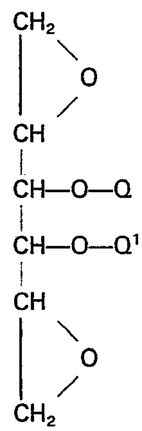

(IX)

(wherein the hexitol skeleton corresponds to dulcitol, mannitol or iditol, Q represents an aralkoxycarbonyl-containing $C_{3-9}$ alkylcarbonyl or alkenylcarbonyl group and Q represents a hydrogen atom, a $C_{2-10}$ alkylcarbonyl or alkenylcarbonyl group, an alkoxycarbonyl-containing alkylcarbonyl or alkenylcarbonyl group with 4 to 10 carbon atoms, an aralkoxycarbonyl-containing $C_{3-9}$ alkylcarbonyl or alkenylcarbonyl group or a $C_{8-10}$ aralkylcarbonyl or aralkenylcarbonyl group) in the presence of a hydrogenation catalyst substantially ineffective to cause cleavage of the oxirane rings; or

(b) for the preparation of a hexitol wherein $R^2$ and $R^3$ together form an oxygen atom, reacting a dulcitol, mannitol or iditol derivative of the formula (X):

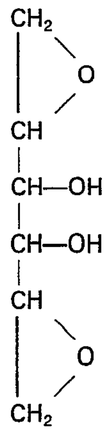

(X)

with a compound of the formula (XI):

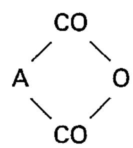

(XI)

wherein A represents a $C_{2-10}$ alkyl, aralkyl or aryl group; or

(c) for the preparation of a hexitol wherein $R^2$ represents a halogen atom and $R^3$ represents a hydroxy group, reacting a hexitol of general formula (I) wherein $R^2$ and $R^3$ together form an oxygen atom with a halide.

2. A process as claimed in claim 1, part (a), wherein the hydrogenation catalyst comprises a palladium/carbon catalyst and wherein said hydrogenating is effected in the presence of an anhydrous medium.

3. A process as claimed in claim 1, part (c), wherein the halide comprises an alkali metal halide and/or a hydrogen halide.

4. A process as claimed in claim 1 or 3 wherein $R^2$ represents a bromine atom and $R^3$ represents a hydroxy group.

5. A process as claimed in claim 1 or 2 wherein $R^2$ and $R^3$ together form an oxygen atom.

6. A process as claimed in claim 5 wherein the product is 1,2 - 5,6 - dianhydro - 3 - (β - carboxypropionyl) - dulcitol or a salt thereof.

7. A process as claimed in claim 5 wherein the product is 1,2 - 5,6 - dianhydro - 3 - (β - carboxypropionyl) - 4 - acetyl - dulcitol or a salt thereof.

8. A process as claimed in claim 5 wherein the product is 1,2 - 5,6 - dianhydro - 3 - (β - carboxypropionyl) - 4 - (β - carbomethoxypropionyl) - dulcitol or a salt thereof.

9. A process as claimed in claim 5 wherein the product is 1,2 - 5,6 - dianhydro - 3 - (β - carboxypropionyl) - 4 - (β - phenylpropionyl) - dulcitol or a salt thereof.

10. A process as claimed in claim 1, part (c) wherein the product is 1,6 - dideoxy - 1,6 - dibromo - 3,4 - bis(β - carboxypropionyl) - dulcitol or a salt thereof.

11. A process as claimed in claim 5 wherein the product is 1,2 - 5,6 - dianhydro - 3 - (β - carboxypropionyl) - mannitol or a salt thereof.

12. A process as claimed in claim 5 wherein the product is 1,2 - 5,6 - dianhydro - 3,4 - bis(β - carboxypropionyl) - mannitol or a salt thereof.

13. A process as claimed in claim 5 wherein the product is 1,2 - 5,6 - dianhydro - 3,4 - bis(β - carboxypropionyl) - dulcitol or a salt thereof.

14. The use of a hexitol of general formula (I) as defined in any of claims 1 to 13 or a physiologically acceptable salt thereof for the manufacture of a medicament for cytostatic or anti-tumour therapy.

15. The use of 1,2 - 5,6 - dianhydro - 3,4 - bis(β - carboxypropionyl) - dulcitol or a physiologically compatible salt for the manufacture of a medicament for cytostatic or anti-tumour therapy.

**Patentansprüche für die Vertragsstaaten: BE, CH, DE, FR, GB, IT, LI, NL, SE**

1. Ein Hexit der allgemeinen Formel (I)

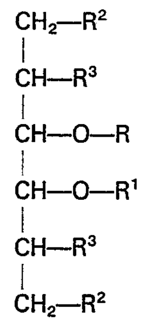

(I),

worin das Hexitskelett dem Dulcit, Mannit oder Idit entspricht,

$R^2$ ein Halogenatom,

$R^3$ eine Hydroxygruppe bedeuten oder

$R^2$ und $R^3$ gemeinsam ein Sauerstoffatom bilden,

R eine freies Carboxyl-enthaltende Alkylcarbonyl- oder Alkenylcarbonylgruppe mit 4—10 C-Atomen, eine freies Carboxylenthaltende Arylcarbonylgruppe mit 8—12 C-Atomen oder eine freies Carboxyl-enthaltende Aralkylcarbonyl- oder Aralkenylcarbonylgruppe mit 9—11 C-Atomen und

$R^1$ ein Wasserstoffatom, eine freies Carboxyl-enthaltende Alkylcarbonyl- oder Alkenylcarbonylgruppe mit 4—10 C-Atomen, eine $C_{2-10}$-Alkylcarbonyl- oder Alkenylcarbonylgruppe, eine Alkoxycarbonyl-enthaltende Alkylcarbonyl- oder Alkenylcarbonylgruppe mit 4—10 C-Atomen, eine $C_{8-10}$-Aralkylcarbonyl- oder Aralkenylcarbonylgruppe, eine freies Carboxyl-enthaltende Arylcarbonylgruppe mit 8—12 C-Atomen oder eine freies Carboxyl-enthaltende Aralkylcarbonyl- oder Aralkenylcarbonylgruppe mit 9—11 C-Atomen bedeuten, oder eines seiner Salze.

2. Hexit gemäß Anspruch 1, worin $R^2$ ein Bromatom und $R^3$ eine Hydroxygruppe bedeuten.

3. Hexit gemäß Anspruch 1, worin $R^2$ und $R^3$ gemeinsam ein Sauerstoffatom bilden.

4. Hexit gemäß Anspruch 1, das 1,2 - 5,6 - Dianhydro - 3 - (β - carboxypropionyl) - dulcit oder eines seiner Salze ist.

5. Hexit gemäß Anspruch 1, das 1,2 - 5,6 - Dianhydro - 3 - (β - carboxypropionyl) - 4 - acetyl - dulcit oder eines seiner Salze ist.

6. Hexit gemäß Anspruch 1, das 1,2 - 5,6 - Dianhydro - 3 - (β - carboxypropionyl) - 4 - (β - carbomethoxypropionyl) - dulcit oder eines seiner Salze ist.

7. Hexit gemäß Anspruch 1, das 1,2 - 5,6 - Dianhydro - 3 - (β - carboxypropionyl) - 4 - (β - phenylpropionyl) - dulcit oder eines seiner Salze ist.

8. Hexit gemäß Anspruch 1, das 1,6 - Dideoxy - 1,6 - dibrom - 3,4 - bis - (β - carboxypropionyl) - dulcit oder eines seiner Salze ist.

9. Hexit gemäß Anspruch 1, das 1,2 - 5,6 - Dianhydro - 3 - (β - carboxypropionyl) - mannit oder eines seiner Salze ist.

10. Hexit gemäß Anspruch 1, das 1,2 - 5,6 - Dianhydro - 3,4 - bis - (β - carboxypropionyl) - mannit oder eines seiner Salze ist.

11. Hexit gemäß Anspruch 1, das 1,2 - 5,6 - Dianhydro - 3,4 - bis - (β - carboxypropionyl) - dulcit oder eines seiner Salze ist.

12. Pharmazeutische Komposition, dadurch gekennzeichnet, daß sie als aktives Ingrediens ein Hexit der Formel I, gemäß der Definition der Ansprüche 1 bis 11 oder eines seiner physiologisch verträglichen Salze in Kombination mit einem pharmazeutischen Trägermaterial oder einem Excipienten enthält.

13. Pharmazeutische Komposition, dadurch gekennzeichnet, daß sie als aktives Ingrediens 1,2 - 5,6 - Dianhydro - 3,4 - bis - (β - carboxypropionyl) - dulcit oder eines seiner physiologisch verträglichen Salze in Kombination mit einem pharmazeutischen Trägermaterial oder Excipienten enthält.

14. Verfahren zur Herstellung eines Hexits gemäß Anspruch 3, dadurch gekennzeichnet, daß eine Verbindung der Formel (IX)

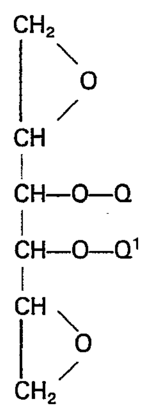

(IX),

worin das Hexitskelett dem Dulcit, Mannit oder Idit entspricht, Q eine Aralkoxycarbonyl-enthaltende $C_{3-9}$-Alkylcarbonyl- oder Alkenylcarbonylgruppe und $Q^1$ ein Wasserstoffatom, eine $C_{2-10}$-Alkylcarbonyl- oder Alkenylcarbonylgruppe, eine Alkoxycarbonyl-enthaltende Alkylcarbonyl- oder Alkenylcarbonylgruppe mit 4—10 C-Atomen, eine Aralkoxycarbonyl-enthaltende $C_{3-9}$-Alkylcarbonyl- oder Alkenylcarbonylgruppe oder eine $C_{8-10}$-Aralkylcarbonyl- oder Alkenylcarbonylgruppe bedeuten, in Gegenwart eines Hydrierungskatalysators, der praktisch zu unwirksam ist, um die Spaltung von Oxiranringen zu bewirken, hydriert, oder ein Dulcit-, Mannit- oder Idit-derivat der Formel (X)

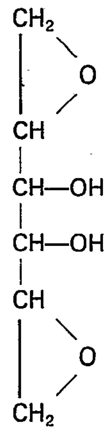

(X)

mit einer Verbindung der Formel (XI)

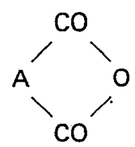

(XI),

worin A eine $C_{2-10}$-Alkyl-, Aralkyl- oder Arylgruppe bedeutet, umgesetzt wird.

15. Verfahren nach Anspruch 14, dadurch gekennzeichnet, daß als Hydrierungskatalysator ein Palladium/Kohlekatalysator verwendet wird.

16. Verfahren nach Anspruch 14, dadurch gekennzeichnet, daß die Hydrierung oder Umsetzung in Gegenwart eines wasserfreien Mediums durchgeführt wird.

17. Verfahren zur Herstellung eines Hexits gemäß Anspruch 1, worin $R^2$ ein Halogenatom und $R^3$ eine Hydroxygruppe bedeuten, dadurch gekennzeichnet, daß ein Hexit gemäß Anspruch 3 mit einem Halogenid umgesetzt wird.

18. Verfahren nach Anspruch 17, dadurch gekennzeichnet, daß als Halogenid ein Alkalimetallhalogenid und/oder ein Halogenwasserstoff verwendet wird.

**Patentansprüche für den Vertragsstaat: AT**

1. Verfahren zur Herstellung eines Hexits der allgemeinen Formel (I)

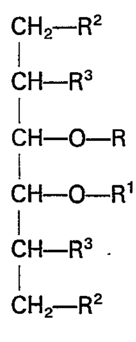

(I),

worin das Hexitsskelett dem Dulcit, Mannit oder Idit entspricht,

$R^2$ ein Halogenatom,

$R^3$ eine Hydroxygruppe bedeuten oder

$R^2$ und $R^3$ gemeinsam ein Sauerstoffatom bilden,

R eine freies Carboxyl-enthaltende Alkylcarbonyl- oder Alkenylcarbonylgruppe mit 4—10 C-Atomen, eine freies Carboxyl-enthaltende Arylcarbonylgruppe mit 8—12 C-Atomen oder eine freies Carboxyl-enthaltende Aralkylcarbonyl- oder Aralkenylcarbonylgruppe mit 9—11 C-Atomen und

$R^1$ ein Wasserstoffatom, eine freies Carboxyl-enthaltende Alkylcarbonyl- oder Alkenylcarbonylgruppe mit 4—10 C-Atomen, eine $C_{2-10}$-Alkylcarbonyl- oder Alkenylcarbonylgruppe, eine Alkoxycarbonyl-enthaltende Alkylcarbonyl- oder Alkenylcarbonylgruppe mit 4—10 C-Atomen, eine $C_{8-10}$-Aralkylcarbonyl- oder Aralkenylcarbonylgruppe, eine freies Carboxylenthaltende Arylcarbonylgruppe mit 8—12 C-Atomen oder eine freies Carboxyl-enthaltende Aralkylcarbonyl- oder Aralkenylcarbonylgruppe mit 9—11 C-Atomen bedeuten, oder eines seiner Salze, dadurch gekennzeichnet, daß

a) eine Verbindung der Formel (IX)

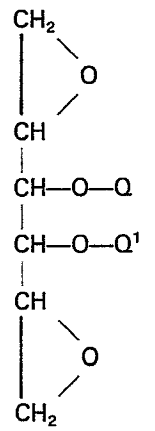

(IX),

worin das Hexitskelett dem Dulcit, Mannit oder Idit entspricht, Q eine Aralkoxycarbonyl-enthaltende $C_{3-9}$-Alkylcarbonyl- oder Alkenylcarbonylgruppe und $Q^1$ ein Wasserstoffatom, eine $C_{2-10}$-Alkylcarbonyl- oder Alkenylcarbonylgruppe, eine Alkoxycarbonyl-enthaltende Alkylcarbonyl- oder Alkenylcarbonylgruppe mit 4—10 C-Atomen, eine Aralkoxycarbonyl-enthaltende $C_{3-9}$-Alkylcarbonyl- oder Alkenylcarbonylgruppe oder eine $C_{8-10}$-Aralkylcarbonyl- oder Alkenylcarbonylgruppe bedeuten, in Gegenwart eines Hydrierungskatalysators, der praktisch zu unwirksam ist, um die Spaltung von Oxiranringen zu bewirken, hydriert, oder

b) ein Dulcit-, Mannit- oder Idit-derivat der Formel (X)

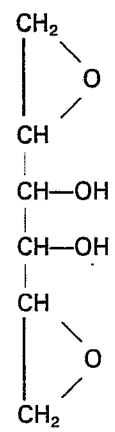

(X)

mit einer Verbindung der Formel (XI)

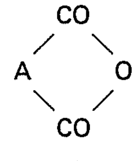

(XI),

worin A eine $C_{2-10}$-Alkyl-, Aralkyl- oder Arylgruppe bedeutet, umgesetzt, oder

c) zur Herstellung eines Hexits, worin $R^2$ ein Halogenatom und $R^3$ eine Hydroxygruppe bedeuten, ein Hexit der allgemeinen Formel (I), worin $R^2$ und $R^3$ gemeinsam ein Sauerstoffatom bilden, mit einem Halogenid umgesetzt wird.

2. Verfahren nach Anspruch 1, Teil a), dadurch gekennzeichnet, daß als Hydrierungskatalysator ein Palldium/Kohlekatalysator verwendet und die Hydrierung in Gegenwart eines wasserfreien Mediums durchgeführt wird.

3. Verfahren nach Anspruch 1, Teil c) dadurch gekennzeichnet, daß als Halogenid ein Alkalimetallhalogenid und/oder ein Halogenwasserstoff verwendet wird.

4. Verfahren nach Anspruch 1 oder 3, dadurch gekennzeichnet, daß $R^2$ ein Bromatom und $R^3$ eine Hydroxygruppe ist.

5. Verfahren nach Anspruch 1 oder 2, dadurch gekennzeichnet, daß $R^2$ und $R^3$ gemeinsam ein Sauerstoffatom bilden.

6. Verfahren nach Anspruch 5, dadurch gekennzeichnet, daß das Produkt 1,2 - 5,6 - Dianhydro - 3 - (β - carboxypropionyl) - dulcit oder eines seiner Salze ist.

7. Verfahren nach Anspruch 5, dadurch gekennzeichnet, daß das Produkt, 1,2 - 5,6 - Dianhydro - 3 - (β - carboxypropionyl) - 4 - acetyl - dulcit oder eines seiner Salze ist.

8. Verfahren nach Anspruch 5, dadurch gekennzeichnet, daß das Produkt 1,2 - 5,6 - Dianhydro - 3 - (β - carboxypropionyl) - 4 - (β - carbomethoxypropionyl) - dulcit oder eines seiner Salze ist.

9. Verfahren nach Anspruch 5, dadurch gekennzeichnet, daß das Produkt 1,2 - 5,6 - Dianhydro - 3 - (β - carboxypropionyl) - 4 - (β - phenylpropionyl) - dulcit oder eines seiner Salze ist.

10. Verfahren nach Anspruch 5, dadurch gekennzeichnet, daß das Produkt 1,6 - Dideoxy - 1,6 - dibrom - 3,4 - bis - (β - carboxypropionyl) - dulcit oder eines seiner Salze ist.

11. Verfahren nach Anspruch 5, dadurch gekennzeichnet, daß das Produkt 1,2 - 5,6 - Dianhydro - 3 - (β - carboxypropionyl) - mannit oder eines seiner Salze ist.

12. Verfahren nach Anspruch 5, dadurch gekennzeichnet, daß das Produkt 1,2 - 5,6 - Dianhydro - 3,4 - bis - (β - carboxypropionyl) - mannit oder eines seiner Salze ist.

13. Verfahren nach Anspruch 5, dadurch gekennzeichnet, daß das Produkt 1,2 - 5,6 - Dianhydro - 3,4 - bis - (β - carboxypropionyl) - dulcit oder eines seiner Salze ist.

14. Verwendung eines Hexits der allgemeinen Formel I gemäß der Difinition einer der Ansprüche 1 bis 13 oder eines seiner physiologisch verträglichen Salze zur Herstellung eines pharmazeutischen Präparates für die cytostatische oder die Anti-tumor-Therapie.

15. Verwendung von 1,2 - 5,6 - Dianhydro - 3,4 - bis - (β - carboxypropionyl) - dulcit oder eines seiner physiologisch verträglichen Salze zur Herstellung eines pharmazeutischen Präparates für die cytostatische oder die Anti-tumor-Therapie.

**Revendications pour les Etats Contractants: BE, CH, DE, FR, GB, IT, LI, NL, SE**

1. Hexitol de formule générale (I)

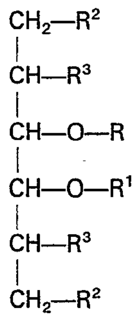

(I)

(où le squelette hexitol correspond à dulcitol, mannitol ou iditol;

$R^2$ représente un atome d'halogène,

$R^3$ représente un groupe hydroxy, ou

$R^2$ et $R^3$ forment ensemble un atome d'oxygène;

R représente un groupe alcoylcarbonyle ou alcénylcarbonyle en C8 à C12 contenant un carboxy libre ou un groupe aralcoylcarbonyle ou aralcénylcarbonyle en C9 à C11 contenant un carboxy libre; et

$R^1$ représente un atome d'hydrogène, un groupe alcoylcarbonyle ou alcénylcarbonyle en C4 à C10 contenant un carboxy libre, un groupe alcoylcarbonyle ou alcénylcarbonyle en C2 à C10, un groupe alcoylcarbonyle ou alcénylcarbonyle en C4 à C10 contenant un alcoxycarbonyle, un groupe aralcoylcarbonyle ou aralcénylcarbonyle en C8 à C10, un groupe arylcarbonyle en C8 à C12 contenant un carboxy libre ou un groupe aralcoylcarbonyle ou aralcénylcarbonyle en C9 à C11 contenant un carboxy libre) ou un de ses sels.

2. Hexitol selon la revendication 1 où $R^2$ représente un atome de brome et $R^3$ représente un groupe hydroxy.

3. Hexitol selon la revendication 1 où $R^2$ et $R^3$ forment ensemble un atome d'oxygène.

4. Hexitol selon la revendication 1 qui est le 1,2 - 5,6 - dianhydro - 3 - (β - carboxypropionyl) - dulcitol ou un de ses sels.

5. Hexitol selon la revendication 1 qui est le 1,2 - 5,6 - dianhydro - 3 - (β - carboxypropionyl) - 4 - acétyl - dulcitol ou un de ses sels.

6. Hexitol selon la revendication 1 qui est le 1,2 - 5,6 - dianhydro - 3 - (β - carboxypropionyl) - 4 - (β - carbométhoxypropionyl) - dulcitol ou un de ses sels.

7. Hexitol selon la revendication 1 qui est le 1,2 - 5,6 - dianhydro - 3 - (β - carboxypropionyl) - 4 - (β - phénylpropionyl) - dulcitol ou un de ses sels.

8. Hexitol selon la revendication 1 qui est le 1,6 - didésoxy - 1,6 - dibromo - 3,4 - bis(β - carboxypropionyl) - dulcitol ou un de ses sels.

9. Hexitol selon la revendication 1 qui est le 1,2 - 5,6 - dianhydro - 3 - (β - carboxypropionyl) - mannitol ou un de ses sels.

10. Hexitol selon la revendication 1 qui est le 1,2 - 5,6 - dianhydro - 3,4 - bis(β - carboxypropionyl) - mannitol ou un de ses sels.

11. Hexitol selon la revendication 1 qui est le 1,2 - 5,6 - dianhydro - 3,4 - bis(β - carboxypropionyl) - dulcitol ou un de ses sels.

12. Composition pharmaceutique comprenant comme ingrédient actif un hexitol de formule I tel que défini dans l'une quelconque des revendications 1 à 11 ou un de ses sels physiologiquement compatibles en association avec un support ou excipient pharmaceutique.

13. Composition pharmaceutique comprenant comme ingrédient pharmaceutique le 1,2 - 5,6 - dianhydro - 3,4 - bis(β - carboxypropionyl) - dulcitol ou un de ses sels physiologiquement compatibles en association avec un support ou excipient pharmaceutique.

14. Procédé de préparation d'un hexitol tel que défini dans la revendication 3, dans lequel on hydrogène un composé de formule (IX):

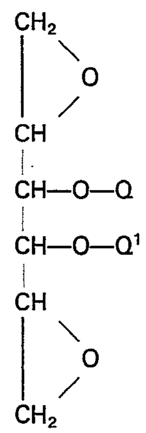

(IX)

(où le squelette hexitol correspond à dulcitol, mannitol ou iditol, Q représente un groupe alcoylcarbonyle ou alcénylcarbonyle en C3 à C9 contenant un aralcoxycarbonyle et $Q^1$ représente un atome d'hydrogène, un groupe alcoylcarbonyle ou alcénylcarbonyle en C2 à C10, un groupe alcoylcarbonyle ou alcénylcarbonyle en C4 à C10 contenant un alcoxycarbonyle, un groupe alcoylcarbonyle ou alcénylcarbonyle en C3 à C9 contenant un aralcoxycarbonyle ou un groupe aralcoylcarbonyle ou alcénylcarbonyle en C8 à C10) en présence d'un catalyseur d'hydrogénation essentiellement inefficace pour provoquer la rupture des noyaux oxiranne; ou on fait réagir un dérivé de dulcitol, de mannitol ou d'iditol de formule (X):

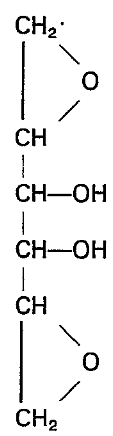

(X)

avec un composé de formule (XI):

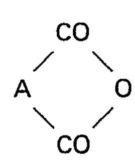

(XI)

où A représente un groupe alcoyle, aralcoyle ou aryle en C2 à C10.

15. Procédé selon la revendication 14 où le catalyseur d'hydrogénation comprend un catalyseur palladium/charbon.

16. Procédé selon la revendication 14 où ladite hydrogénation ou réaction est conduite en présence d'un milieu anhydre.

17. Procédé de préparation d'un hexitol tel que défini dans la revendication 1 où $R^2$ représente un atome d'halogène et $R^3$ représente un groupe hydroxy, dans lequel on fait réagir un hexitol tel que défini dans la revendication 3 avec un halogénure.

18. Procédé selon la revendication 17 où l'halogénure comprend un halogénure de métal alcalin et/ou un acide halohydrique.

**Revendications pour l'Etat Contractant: AT**

1. Procédé de préparation d'un hexitol de formule générale (I)

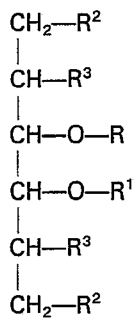

(I)

(où le squelette hexitol correspond à dulcitol, mannitol ou iditol;

$R^2$ représente un atome d'halogène,

$R^3$ représente un groupe hydroxy, ou

$R^2$ et $R^3$ forment ensemble un atome d'oxygène;

R représente un groupe alcoylcarbonyle ou alcénylcarbonyle en C4 à C10 contenant un carboxy libre, un groupe arylcarbonyle en C8 à C12 contenant un carboxy libre ou un groupe aralcoylcarbonyle ou aralcénylcarbonyle en C9 à C11 contenant un carboxy libre; et

$R^1$ représente un atome d'hydrogène, un groupe alcoylcarbonyle ou alcénylcarbonyle en C4 à C10 contenant un carboxy libre, un groupe alcoylcarbonyle ou alcénylcarbonyle en C2 à C10, un groupe alcoylcarbonyle ou alcénylcarbonyle en C4 à C10 contenant un alcoxycarbonyle, un groupe aralcoylcarbonyle ou aralcénylcarbonyle en C8 à C10, un groupe arylcarbonyle en C8 à C12 contenant un carboxy libre ou un groupe aralcoylcarbonyle ou aralcénylcarbonyle en C9 à C11 contenant un carboxy libre) ou un de ses sels; dans lequel

(a) on hydrogène un composé de formule (IX):

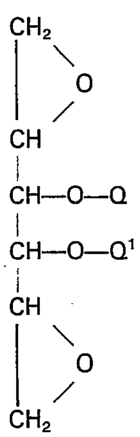

(IX)

(où le squelette hexitol correspond à dulcitol, mannitol ou iditol, Q représente un groupe alcoylcarbonyle ou alcénylcarbonyle en C3 à C9 contenant un aralcoxycarbonyle et $Q^1$ représente un atome d'hydrogène, un groupe alcoylcarbonyle ou alcénylcarbonyle en C2 à C10, un groupe alcoylcarbonyle ou alcénylcarbonyle en C4 à C10 contenant un alcoxycarbonyle, un groupe alcoylcarbonyle ou alcénylcarbonyle en C3 à C9 contenant un aralcoxycarbonyle ou un groupe aralcoylcarbonyle ou alcénylcarbonyle en C8 à C10) en présence d'un catalyseur d'hydrogénation essentiellement inefficace pour provoquer la rupture des noyaux oxiranne ou

(b) on fait réagir un dérivé de dulcitol, de mannitol ou d'iditol de formule (X):

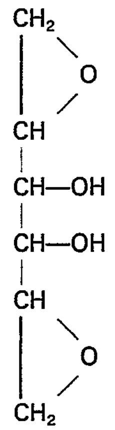

(X)

avec un composé de formule (XI):

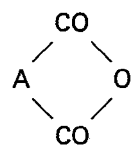

(XI)

où A représente un groupe alcoyle, aralcoyle ou aryle en C2 à C10; ou

(c) pour la préparation d'un hexitol où $R^2$ représente un atome d'halogène et $R^3$ représente un groupe hydroxy, on fait réagir un hexitol de formule générale (I) où $R^2$ et $R^3$ forment ensemble un atome d'oxygène avec un halogénure.

2. Procédé selon la revendication 1, partie (a), où le catalyseur d'hydrogénation comprend un catalyseur palladium/charbon et où ladite hydrogénation est conduite en présence d'un milieu anhydre.

3. Procédé selon la revendication 1, partie (c), où l'halogénure comprend un halogénure de métal alcalin et/ou un acide halohydrique.

4. Procédé selon les revendications 1 ou 3 où $R^2$ représente un atome de brome et $R^3$ représente un groupe hydroxy.

5. Procédé selon les revendications 1 ou 2 où $R^2$ et $R^3$ ensemble forment un atome d'oxygène.

6. Procédé selon la revendication 5 où le produit est le 1,2 - 5,6 - dianhydro - 3 - (β - carboxypropionyl) - dulcitol ou un de ses sels.

7. Procédé selon la revendication 5 où le produit est le 1,2 - 5,6 - dianhydro - 3 - (β - carboxypropionyl) - 4 - acétyl - dulcitol ou un de ses sels.

8. Procédé selon la revendication 5 où le produit est le 1,2 - 5,6 - dianhydro - 3 - (β - carboxypropionyl) - 4 - (β - carbométhoxypropionyl) - dulcitol ou un de ses sels.

9. Procédé selon la revendication 5 où le produit est le 1,2 - 5,6 - dianhydro - 3 - (β - carboxypropionyl) - 4 - (β - phénylpropionyl) - dulcitol ou un de ses sels.

10. Procédé selon la revendication 5 où le produit est le 1,6 - didésoxy - 1,6 - dibromo - 3,4 - bis(β - carboxypropionyl) - dulcitol ou un de ses sels.

11. Procédé selon la revendication 5 où le produit est le 1,2 - 5,6 - dianhydro - 3 - (β - carboxypropionyl) - mannitol ou un de ses sels.

12. Procédé selon la revendication 5 où le produit est le 1,2 - 5,6 - dianhydro - 3,4 - bis(β - carboxypropionyl) - mannitol ou un de ses sels.

13. Procédé selon la revendication 5 où le produit est le 1,2 - 5,6 - dianhydro - 3,4 - bis(β - carboxypropionyl) - dulcitol ou un de ses sels.

14. Application d'un hexitol de formule générale (I) tel que défini dans l'une quelconque des revendications 1 à 13 ou d'un de ses sels physiologiquement acceptables à la préparation d'un médicament pour le traitement cytostatique ou anti-tumoral.

15. Application de 1,2 - 5,6 - dianhydro - 3,4 - bis(β - carboxypropionyl) - dulcitol ou d'un de ses sels physiologiquement compatibles à la preparation d'un médicament pour le traitement cytostatique ou anti-tumoral.